# EUROPEAN PATENT SPECIFICATION

(11) **EP 3 515 210 B1**
(45) Date of publication and mention of the grant of the patent: **04.11.2020**
(21) Application number: 17851374.3
(22) Date of filing: 11.09.2017
(51) Int. Cl.: C08B 37/00, C08L 5/14, A61P 3/00, A61K 36/81, A23L 33/105

(54) **MATERIALS AND METHODS FOR PRODUCING AND USING MITOCHONDRIAL PREPARATIONS**
MATERIALIEN UND VERFAHREN ZUR HERSTELLUNG UND VERWENDUNG VON MITOCHONDRIALEN PRÄPARATEN
MATÉRIAUX ET PROCÉDÉS POUR PRODUIRE ET UTILISER DES PRÉPARATIONS MITOCHONDRIALES

(30) Priority: 19.09.2016 US 201662396521 P
(43) Date of publication of application: 31.07.2019
(73) Proprietor: Mitogenetics, LLC, Farmingdale, New York 11735 (US)
(72) Inventor: KREAM, Richard, Huntington, New York 11743 (US); STEFANO, George B., Melville, New York 11747 (US)
(74) Representative: Fish & Richardson P.C.
(86) International application number: PCT/US2017/050986
(87) International publication number: WO 2018/052849

(56) References cited:
- WO-A1-2013/148282
- WO-A1-2015/041837
- US-A1- 2011 008 310
- US-A1- 2014 193 511

## Description

### BACKGROUND

### 1. Technical Field

This document relates to methods and materials for producing and/or using mitochondrial preparations. For example, this document relates to methods and materials for using xyloglucan (e.g., xyloglucan material obtained from potato) to produce improved mitochondrial preparations. In some cases, mitochondrial preparations provided herein can be administered to cells to improve cellular function and/or to reduce a symptom of a disease or disorder.

### 2. Background Information

The etiology and persistence of major metabolic disorders afflicting millions of humans worldwide involve pathophysiological coupling of systemic pro-inflammatory processes and ischemic reductions of normal oxygen delivery to multiple organ systems. Reciprocal triggering of multiple ischemic/hypoxic and pro-inflammatory events, if not corrected, may converge to impair mitochondrial ATP production markedly with resultant metabolic rundown and loss of tissue and whole organ integrity. Chronic impairment of mitochondrial bioenergetics may severely compromise normative biochemical and molecular processes responsible for ongoing gene expression of the mitochondrial proteome leading to markedly impaired mitochondrial functioning and biogenesis.

WO 2013/148282 A1 describes nutritional supplement compositions containing a potato polysaccharide preparation.

WO 2015/041837 A1 describes compositions containing a potato polysaccharide preparation for use in reducing symptoms of diabetes or liver steatosis.

US 2014/193511 A1 describes compositions of partially purified functional mitochondria.

US 2011/008310 A1 describes compositions for mitochondrial replacement for use in the treatment of disorders arising from mitochondrial dysfunction.

### SUMMARY

The present invention is defined by independent claim 1. The dependent claims depict additional embodiments of the invention.

This document provides methods and materials for producing mitochondrial preparations as well as methods and materials for using mitochondrial preparations. For example, this document provides methods and materials for using xyloglucan (e.g., xyloglucan material obtained from potato) to produce improved mitochondrial preparations. In some cases, mitochondrial preparations provided herein can be administered to cells to improve cellular function and/or to reduce a symptom of a disease or disorder.

As described herein, cells (e.g., blood cells) can be incubated with or placed in contact with a xyloglucan preparation under conditions that improve the activity of mitochondria within those cells. For example, cells (e.g., human cells) can be treated with a xyloglucan preparation provided herein to increase the expression of mitochondrial respiratory complex 1 (NADH:ubiquinone oxidoreductase, EC 1.6.5.3) polypeptides and/or mitochondrial trafficking polypeptides, as compared to those expression levels observed in comparable untreated cells. In some cases, cells (e.g., human cells) can be treated with a xyloglucan preparation provided herein to increase the activity of mitochondria within the cells, as compared to that observed in comparable untreated cells. In some cases, an increase in mitochondrial activity can be assessed using a probe (e.g., a dye or stain) capable of assessing mitochondrial activity (e.g., rhodamine 6G).

In some cases, a mitochondrial preparation can be prepared from cells treated with a xyloglucan preparation provided herein. For example, mitochondria can be isolated from cells treated with a xyloglucan preparation to create a mitochondrial preparation. The mitochondria of such a mitochondrial preparation can have an increased level of expression of mitochondrial respiratory complex 1 polypeptides, an increased level of expression of mitochondrial trafficking polypeptides, and/or an increased level of mitochondrial activity, as compared to those observed in mitochondria obtained from comparable untreated cells (i.e., comparable cells not treated with a xyloglucan preparation).

In some cases, a mitochondrial preparation provided herein can be administered to a mammal. The administered mitochondria can provide tissues and cells of the mammal with several benefits both when present extracellularly and intracellularly within the mammal. For example, when administered to a mammal and present extracellularly within that mammal, the intact and viable mitochondria administered to the mammal as described herein can provide cells and tissue with ATP and can stimulate the expression of polypeptides involved mitochondrial pathways. When administered to a mammal and internalized into cells within that mammal, the intact and viable mitochondria administered to the mammal as described herein can provide those cells with increased ATP production and a source of new, exogenously added mitochondrial DNA.

Having the ability to produce mitochondrial preparations having intact and viable mitochondria isolated from cells exposed a xyloglucan preparation as described herein allows clinicians to not only obtain mitochondria with improved activity but also to administer mitochondria with improved activity to mammals. Administering mitochondria with improved activity to mammals suffering from a disease or disorder involving impaired or defective mitochondrial function as described herein can allow those mammals to experience a reduced severity of a symptom of that disease or disorder.

In general, one aspect of this document features a composition comprising potato xyloglucan material and viable mitochondria separated from cells. The potato xyloglucan material can be material obtained from raw potato. The cells can be mammalian cells. The cells can be human cells. The composition can comprise organic selenium. The composition can comprise an inorganic nitrate.

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting. In case of conflict between the present specification and a reference mentioned herein, the present specification, including definitions, will control.

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure 1 contains photographs of control HTB-11 cells (right panel) and HTB-11 cells treated with xyloglucan for 24 hours (left panel) and stained with rhodamine 6G for 30 minutes.
Figure 2 is a photograph of HTB-11 cells treated with xyloglucan for 24 hours and stained with rhodamine 6G for 30 minutes. Arrows indicate the presence of fluorescent mitochondria within tunneling nanotubes.

### DETAILED DESCRIPTION

This document provides methods and materials for producing mitochondrial preparations. For example, this document provides methods and materials for using xyloglucan (e.g., xyloglucan material obtained from potato) to produce improved mitochondrial preparations. As described herein, cells can be exposed to a xyloglucan preparation to increase the level of expression of mitochondrial respiratory complex 1 polypeptides, to increase the level of expression of mitochondrial trafficking polypeptides, and/or to increase the level of mitochondrial activity, thereby generating mitochondria within those treated cells that are more active than the mitochondria present in comparable cells not treated with the xyloglucan preparation.

In general, a xyloglucan preparation provided herein can include a xyloglucan preparation obtained from any appropriate potato. For example, a xyloglucan preparation provided herein can be a potato polysaccharide preparation over 50 percent (e.g., over 60, 70, 80, 90, 95, or 99 percent) of which is xyloglucan obtained from one or more raw potatoes. In some cases, the xyloglucan material of a xyloglucan preparation provided herein can be highly substituted complex xyloglucan material and/or can be heterogeneous in nature.

In some cases, a xyloglucan preparation provided herein can be designed to include potato xyloglucans that are obtained from potatoes (e.g. raw potatoes) and that, when derivatized and assessed using GC/MS, result in at least four major components (3,4-furan dimethanol, diacetate; 1,2,3,4,5-penta-o-acetyl-D-xylitol (isomer 1); 3,5-diacetoxybenzyl alcohol; and D-glucitol-hexaacetate). In some cases, a xyloglucan preparation provided herein can be a xyloglucan preparation that is obtained from potatoes (e.g., raw potatoes) and that, when derivatized, results in at least the following acylated carbohydrates as assessed using GC/MS: (a) myo-inositol (set to 1X to serve as an internal standard), (b) glucose at about 40X to about 60X the myo-inositol content (e.g., glucose at about 50X the myo-inositol content), (c) xylose at about 10X to about 20X the myo-inositol content (e.g., xylose at about 15X the myo-inositol content), (d) mannose at about 5X to about 15X the myo-inositol content (e.g., mannose at about 10X the myo-inositol content), and (e) galactose at about 3X to about 7X the myo-inositol content (e.g., galactose at about 5X the myo-inositol content). The derivatization procedure can include forming a dry residue of the xyloglucan material that is then hydrolyzed using trifluoroacetic acid. The resulting material is then reduced using sodium borohydride, and after borate removal, the end product is acylated using acetic anhydride and pyridine. The end products of the reaction are then injected directly on GC/MS to identify the acylated carbohydrates.

In some cases, a xyloglucan preparation provided herein can be a substantially pure xyloglucan preparation. Typically, a substantially pure xyloglucan preparation is a preparation that contains a single peak of material (*e.g.,* a single peak of xyloglucan material) when assessed using, for example, HPLC. In some cases, greater than 60, 70, 75, 80, 85, 90, 95, or 99 percent of a xyloglucan preparation provided herein can be xyloglucan material obtained from a potato. In some cases, the xyloglucan material of a xyloglucan preparation provided herein can be a polar, water-soluble xyloglucan.

Any appropriate method can be used to obtain a xyloglucan preparation for treating cells as described herein. For example, a xyloglucan preparation can be a preparation that is obtained from a water extract of potato and that contains xyloglucan material having the ability to be eluted from a C18 cartridge (*e.g.,* a Sep-Pak Plus C-18 cartridge) with 10% acetonitrile. In some cases, a xyloglucan preparation can be a preparation that is obtained from potato and that contains xyloglucan material having HPLC characteristics of that of the peak eluted at 3.5 minutes as described elsewhere (e.g., International Patent Application Publication No. WO 2013/148282). In some cases, the methods described elsewhere for making a potato polysaccharide preparation (e.g., International Patent Application Publication No. WO 2013/148282) can be used to make a xyloglucan preparation that can be used as described herein.

In some cases, a xyloglucan preparation can be obtained by homogenizing raw potato material in water and maintaining the homogenate at room temperature for a period of time (*e.g.,* about 1 hour) with occasional shaking. The homogenate can be centrifuged (*e.g.,* at 4000 g for 10 minutes) to remove any larger solid material. The resulting supernatant can be loaded onto a Solid Phase Extraction cartridge (*e.g.,* a C18 cartridge such as a Sep-Pak Plus C-18 cartridge), and the xyloglucan material eluted with 10 percent acetonitrile. Once eluted, the xyloglucan material can be dried and stored (*e.g.,* at about 4°C).

Any appropriate potato species or variety can be used to obtain a xyloglucan preparation described herein. For example, *Solanum tuberosum, Ipomoea batatas, S. acaule, S. bukasovii, S. leptophyes, S. megistacrolobum, S. commersonii,* or *S. infundibuliforme* can be used to obtain a xyloglucan preparation provided herein. In some cases, potato varieties of *S. tunerosum* such as Organic Yellow, Purple or blue varieties, Cream of the Crop, Adirondack Blue, Adirondack Red, Agata, Almond, Andes Gold, Andes Sun, Apline, Alturas, Amandine, Annabelle, Anya, Arran Victory, Atlantic, Avalanche, Bamberg, Bannock Russet, Belle de Fontenay, BF-15, Bildtstar, Bintje, Blazer Russet, Blue Congo, Bonnotte, British Queens, Cabritas, Camota, Canela Russet, Cara, Carola, Chelina, Chiloe, Cielo, Clavela Blanca, Desiree, Estima, Fianna, Fingerling, Flava, German Butterball, Golden Wonder, Goldrush, Home Guard, Innovator, Irish Cobbler, Jersey Royal, Kennebec, Kerr's Pink, Kestrel, Keuka Gold, King Edward, Kipfler, Lady Balfour, Langlade, Linda, Marcy, Marfona, Maris Piper, Marquis, Megachip, Monalisa, Nicola, Pachacona, Pike, Pink Eye, Pink Fir Apple, Primura, Ranger Russet, Ratte, Record, Red LaSoda, Red Norland, Red Pontiac, Rooster, Russet Burbank, Russet Norkotah, Selma, Shepody, Sieglinde, Silverton Russet, Sirco, Snowden, Spunta, Up to date, Stobrawa, Superior, Vivaldi, Vitelotte, Yellow Finn, or Yukon Gold can be used to obtain a xyloglucan preparation provided herein.

A xyloglucan preparation provided herein can be used to treat any appropriate cell to increase the level of expression of mitochondrial respiratory complex 1 polypeptides, to increase the level of expression of mitochondrial trafficking polypeptides, and/or to increase the level of mitochondrial activity, thereby generating mitochondria within those treated cells that are more active than the mitochondria present in comparable cells not treated with the xyloglucan preparation. For example, blood cells, muscle cells, nerve cells, vascular endothelial cells, cardiovascular cells, adipogenic/adipocyte cells, or kidney cells can be treated with a xyloglucan preparation provided herein to increase the level of expression of mitochondrial respiratory complex 1 polypeptides, to increase the level of expression of mitochondrial trafficking polypeptides, and/or to increase the level of mitochondrial activity. The cells treated with a xyloglucan preparation provided herein can be from any appropriate mammal including, without limitation, rats, mice, dogs, cats, horses, cows, goats, pigs, monkeys, or humans.

The cells can be treated with a xyloglucan preparation provided herein *in vitro* or *in vivo.* For example, cells in tissue culture can be exposed to or incubated with a xyloglucan preparation provided herein to increase the level of expression of mitochondrial respiratory complex 1 polypeptides, to increase the level of expression of mitochondrial trafficking polypeptides, and/or to increase the level of mitochondrial activity. In such cases, the cells can be collected and processed as described herein to obtain a mitochondrial preparation having intact and viable mitochondria that are liberated from cells and that have an elevated level of activity (as compared to comparable mitochondria obtained from cells not exposed to the xyloglucan preparation).

In some cases, a mammal can be administered (e.g., orally administered) a xyloglucan preparation provided herein for a period of time to expose cells *in vivo* to the xyloglucan preparation, thereby increasing the level of expression of mitochondrial respiratory complex 1 polypeptides, increasing the level of expression of mitochondrial trafficking polypeptides, and/or increasing the level of mitochondrial activity. In such cases, cells of the mammal (e.g., blood cells) can be harvested and processed as described herein to obtain a mitochondrial preparation having intact and viable mitochondria that are liberated from cells and that have an elevated level of activity (as compared to comparable mitochondria obtained from cells not exposed to the xyloglucan preparation).

In some cases, the xyloglucan material of a xyloglucan preparation provided herein can be used as obtained from potatoes. For example, xyloglucan material extracted from potatoes can be used to create a xyloglucan preparation without any further manipulation. In some cases, the xyloglucan material of a xyloglucan preparation provided herein can be used as obtained from potatoes and can be the sole active ingredient of the xyloglucan preparation.

Any appropriate amount of a xyloglucan preparation provided herein can be used to increase the level of expression of mitochondrial respiratory complex 1 polypeptides, to increase the level of expression of mitochondrial trafficking polypeptides, and/or to increase the level of mitochondrial activity within cells. For example, when treating cells *in vitro,* the cells can be exposed to a xyloglucan preparation provided herein such that an amount from about 10 µg of xyloglucan material per mL to about 1000 µg of xyloglucan material per mL (e.g., from about 10 µg of xyloglucan material per mL to about 900 µg of xyloglucan material per mL, from about 10 µg of xyloglucan material per mL to about 750 µg of xyloglucan material per mL, from about 10 µg of xyloglucan material per mL to about 500 µg of xyloglucan material per mL, from about 50 µg of xyloglucan material per mL to about 1000 µg of xyloglucan material per mL, from about 100 µg of xyloglucan material per mL to about 1000 µg of xyloglucan material per mL, from about 500 µg of xyloglucan material per mL to about 1000 µg of xyloglucan material per mL, or from about 50 µg of xyloglucan material per mL to about 500 µg of xyloglucan material per mL) is achieved. When treating cells *in vitro,* the cells can be exposed to a xyloglucan preparation provided herein for an appropriate duration. For example, when treating cells *in vitro,* the cells can be exposed to a xyloglucan preparation provided herein for about 12 hours to about 5 days (e.g., for about 12 hours to about 4 days, for about 12 hours to about 3 days, for about 36 hours to about 5 days, or for about 48 hours to about 5 days).

When treating cells *in vivo,* a mammal (e.g., a human) can be administered a xyloglucan preparation provided herein such that an amount from about 1.0 µg of xyloglucan material per kg to about 100 µg of xyloglucan material per kg (e.g., from about 1.0 µg of xyloglucan material per kg to about 80 µg of xyloglucan material per kg, from about 1.0 µg of xyloglucan material per kg to about 60 µg of xyloglucan material per kg, from about 1.0 µg of xyloglucan material per kg to about 50 µg of xyloglucan material per kg, from about 5 µg of xyloglucan material per kg to about 100 µg of xyloglucan material per kg, from about 10 µg of xyloglucan material per kg to about 100 µg of xyloglucan material per kg, from about 10 µg of xyloglucan material per kg to about 75 µg of xyloglucan material per kg, or from about 25 µg of xyloglucan material per kg to about 50 µg of xyloglucan material per kg) is administered to the mammal (e.g., a human). When treating cells *in vivo,* the cells can be exposed to a xyloglucan preparation provided herein for an appropriate duration. For example, when treating cells *in vivo,* the cells can be exposed to a xyloglucan preparation provided herein for about 12 hours to about 5 days (e.g., for about 12 hours to about 4 days, for about 12 hours to about 3 days, for about 36 hours to about 5 days, or for about 48 hours to about 5 days). In some cases, a xyloglucan preparation provided herein can be administered orally to a mammal daily for about 10 days to about 2 months (e.g., for about 10 days to about 1.5 months, for about 10 days to about 1 month, for about 20 days to about 2 months, for about 30 days to about 2 months, or for about 20 days to about 1.5 months), prior to harvesting cells to obtain a mitochondrial preparation.

In some cases, cells can be harvested from a mammal administered a xyloglucan preparation provided herein for a period of time (e.g., several days to several months) and then incubated *in vitro* in the presence of a xyloglucan preparation provided herein for a period of time (e.g., several hours to several days) prior to obtaining a mitochondrial preparation from those cells.

In some cases, a xyloglucan preparation provided herein can be formulated to include one or more other ingredients in addition to xyloglucan material obtained from potatoes. For example, a xyloglucan preparation provided herein can be formulated to include one or more (e.g., one, two, three, four, five, or more) enzyme cofactors targeting mitochondrial complex 1 activity in addition to xyloglucan material obtained from potatoes. Examples of enzyme cofactors targeting mitochondrial complex 1 activity that can be combined with xyloglucan material obtained from potatoes to create a xyloglucan preparation for use as described herein include, without limitation, thiamin, riboflavin, nicotinamide, pyridoxal phosphate, vitamin B12, ubiquinol, inorganic nitrate, 1-arginine, and selenium. When treating cells *in vitro,* the cells can be exposed to a xyloglucan preparation provided herein such that an amount from about 10 µg of xyloglucan material per mL to about 1000 µg of xyloglucan material per mL (e.g., from about 10 µg of xyloglucan material per mL to about 750 µg of xyloglucan material per mL, from about 10 µg of xyloglucan material per mL to about 500 µg of xyloglucan material per mL, from about 50 µg of xyloglucan material per mL to about 1000 µg of xyloglucan material per mL, or from about 25 µg of xyloglucan material per mL to about 750 µg of xyloglucan material per mL) and an amount from about 0.1 µg of an enzyme cofactor per mL to about 100 µg of an enzyme cofactor per mL (e.g., from about 0.1 µg of an enzyme cofactor per mL to about 75 µg of an enzyme cofactor per mL, from about 0.1 µg of an enzyme cofactor per mL to about 50 µg of an enzyme cofactor per mL, from about 0.5 µg of an enzyme cofactor per mL to about 100 µg of an enzyme cofactor per mL, from about 1 µg of an enzyme cofactor per mL to about 100 µg of an enzyme cofactor per mL, or from about 5 µg of an enzyme cofactor per mL to about 100 µg of an enzyme cofactor per mL) is achieved. When treating cells *in vivo,* a mammal (e.g., a human) can be administered a xyloglucan preparation provided herein such that an amount from about 1 µg of xyloglucan material per kg to about 100 µg of xyloglucan material per kg (e.g., from about 1 µg of xyloglucan material per kg to about 75 µg of xyloglucan material per kg, from about 1 µg of xyloglucan material per kg to about 50 µg of xyloglucan material per kg, from about 5 µg of xyloglucan material per kg to about 100 µg of xyloglucan material per kg, from about 10 µg of xyloglucan material per kg to about 100 µg of xyloglucan material per kg, or from about 5 µg of xyloglucan material per kg to about 75 µg of xyloglucan material per kg) and an amount from about 0.01 µg of an enzyme cofactor per kg to about 10 µg of an enzyme cofactor per kg (e.g., from about 0.01 µg of an enzyme cofactor per kg to about 8 µg of an enzyme cofactor per kg, from about 0.01 µg of an enzyme cofactor per kg to about 5 µg of an enzyme cofactor per kg, from about 0.1 µg of an enzyme cofactor per kg to about 10 µg of an enzyme cofactor per kg, from about 1 µg of an enzyme cofactor per kg to about 10 µg of an enzyme cofactor per kg, or from about 0.1 µg of an enzyme cofactor per kg to about 5 µg of an enzyme cofactor per kg) is administered to the mammal (e.g., a human).

In some cases, a xyloglucan preparation provided herein can be formulated to include one or more B complex vitamins in addition to xyloglucan material obtained from potatoes. Examples of B complex vitamins that can be combined with xyloglucan material obtained from potatoes to create a xyloglucan preparation for use as described herein include, without limitation, thiamine, riboflavin, nicotinamide, pyridoxal phosphate, and Vitamin B12. When treating cells *in vitro,* the cells can be exposed to a xyloglucan preparation provided herein such that an amount from about 10 µg of xyloglucan material per mL to about 1000 µg of xyloglucan material per mL (from about 10 µg of xyloglucan material per mL to about 750 µg of xyloglucan material per mL, from about 10 µg of xyloglucan material per mL to about 500 µg of xyloglucan material per mL, from about 50 µg of xyloglucan material per mL to about 1000 µg of xyloglucan material per mL, or from about 25 µg of xyloglucan material per mL to about 750 µg of xyloglucan material per mL) and an amount from about 1 µg of a B complex vitamin per mL to about 100 µg of a B complex vitamin per mL (e.g., from about 1 µg of a B complex vitamin per mL to about 80 µg of a B complex vitamin per mL, from about 1 µg of a B complex vitamin per mL to about 70 µg of a B complex vitamin per mL, from about 5 µg of a B complex vitamin per mL to about 100 µg of a B complex vitamin per mL, from about 10 µg of a B complex vitamin per mL to about 100 µg of a B complex vitamin per mL, or from about 5 µg of a B complex vitamin per mL to about 75 µg of a B complex vitamin per mL) is achieved. When treating cells *in vivo,* a mammal (e.g., a human) can be administered a xyloglucan preparation provided herein such that an amount from about 1 µg of xyloglucan material per kg to about 100 µg of xyloglucan material per kg (e.g., from about 1 µg of xyloglucan material per kg to about 80 µg of xyloglucan material per kg, from about 1 µg of xyloglucan material per kg to about 70 µg of xyloglucan material per kg, from about 5 µg of xyloglucan material per kg to about 100 µg of xyloglucan material per kg, from about 10 µg of xyloglucan material per kg to about 100 µg of xyloglucan material per kg, or from about 5 µg of xyloglucan material per kg to about 75 µg of xyloglucan material per kg) and an amount from about 1 µg of a B complex vitamin per kg to about 100 µg of a B complex vitamin per kg (e.g., from about 1 µg of a B complex vitamin per kg to about 80 µg of a B complex vitamin per kg, from about 1 µg of a B complex vitamin per kg to about 70 µg of a B complex vitamin per kg, from about 5 µg of a B complex vitamin per kg to about 100 µg of a B complex vitamin per kg, from about 10 µg of a B complex vitamin per kg to about 100 µg of a B complex vitamin per kg, or from about 5 µg of a B complex vitamin per kg to about 75 µg of a B complex vitamin per kg) is administered to the mammal (e.g., a human). In some cases, a xyloglucan preparation can be formulated to include xyloglucan material obtained from potatoes in combination with riboflavin, nicotinamide, and pyridoxal phosphate. Such xyloglucan preparations optionally can include one or more enzyme cofactors targeting mitochondrial complex 1 activity.

In some cases, a xyloglucan preparation provided herein can be formulated to include ubiquinol in addition to xyloglucan material obtained from potatoes. For example, a xyloglucan preparation can be formulated to include xyloglucan material obtained from potatoes in combination with ubiquinol. When treating cells *in vitro,* the cells can be exposed to a xyloglucan preparation provided herein such that an amount from about 10 µg of xyloglucan material per mL to about 100 µg of xyloglucan material per mL (e.g., from about 10 µg of xyloglucan material per mL to about 80 µg of xyloglucan material per mL, from about 10 µg of xyloglucan material per mL to about 70 µg of xyloglucan material per mL, from about 15 µg of xyloglucan material per mL to about 100 µg of xyloglucan material per mL, from about 25 µg of xyloglucan material per mL to about 100 µg of xyloglucan material per mL, or from about 25 µg of xyloglucan material per mL to about 75 µg of xyloglucan material per mL) and an amount from about 1 µg of ubiquinol per mL to about 100 µg of ubiquinol per mL (e.g., from about 1 µg of ubiquinol per mL to about 80 µg of ubiquinol per mL, from about 1 µg of ubiquinol per mL to about 70 µg of ubiquinol per mL, from about 5 µg of ubiquinol per mL to about 100 µg of ubiquinol per mL, from about 10 µg of ubiquinol per mL to about 100 µg of ubiquinol per mL, or from about 5 µg of ubiquinol per mL to about 75 µg of ubiquinol per mL) is achieved. When treating cells *in vivo,* a mammal (e.g., a human) can be administered a xyloglucan preparation provided herein such that an amount from about 10 µg of xyloglucan material per kg to about 1000 µg of xyloglucan material per kg (e.g., from about 10 µg of xyloglucan material per kg to about 750 µg of xyloglucan material per kg, from about 10 µg of xyloglucan material per kg to about 500 µg of xyloglucan material per kg, from about 25 µg of xyloglucan material per kg to about 1000 µg of xyloglucan material per kg, from about 50 µg of xyloglucan material per kg to about 1000 µg of xyloglucan material per kg, or from about 50 µg of xyloglucan material per kg to about 750 µg of xyloglucan material per kg) and an amount from about 10 µg of ubiquinol per kg to about 200 µg of ubiquinol per kg (e.g., from about 10 µg of ubiquinol per kg to about 180 µg of ubiquinol per kg, from about 10 µg of ubiquinol per kg to about 170 µg of ubiquinol per kg, from about 25 µg of ubiquinol per kg to about 200 µg of ubiquinol per kg, from about 50 µg of ubiquinol per kg to about 200 µg of ubiquinol per kg, or from about 25 µg of ubiquinol per kg to about 150 µg of ubiquinol per kg) is administered to the mammal (e.g., a human). Such xyloglucan preparations optionally can include one or more enzyme cofactors targeting mitochondrial complex 1 activity and/or one or more B complex vitamins.

In some cases, a xyloglucan preparation provided herein can be formulated to include selenium, inorganic nitrate, arginine, and/or calcium in addition to xyloglucan material obtained from potatoes. Any appropriate amount of organic selenium can be used to formulate a xyloglucan preparation containing xyloglucan material obtained from potatoes. The organic selenium can be a low, non-toxic concentration of L-selenomethionine. For example, a xyloglucan preparation provided herein can contain between about 0.05 nM and about 1 nM (*e.g.,* between about 0.06 nM and about 0.9 nM, between about 0.07 nM and about 0.8 nM, between about 0.08 nM and about 0.7 nM, between about 0.09 nM and about 0.6 nM, between about 0.1 nM and about 0.5 nM, or between about 0.2 nM and about 0.4 nM) of an organic selenium component. When treating cells *in vitro,* the cells can be exposed to a xyloglucan preparation provided herein such that an amount from about 0.005 ng of organic selenium/selenomethionine per mL to about 0.5 ng of organic selenium/selenomethionine per mL (e.g., from about 0.005 ng of organic selenium/selenomethionine per mL to about 0.075 ng of organic selenium/selenomethionine per mL, from about 0.005 ng of organic selenium/selenomethionine per mL to about 0.05 ng of organic selenium/selenomethionine per mL, from about 0.01 ng of organic selenium/selenomethionine per mL to about 0.5 ng of organic selenium/selenomethionine per mL, or from about 0.01 ng of organic selenium/selenomethionine per mL to about 0.025 ng of organic selenium/selenomethionine per mL) is achieved. When treating cells *in vivo,* a mammal (e.g., a human) can be administered a xyloglucan preparation provided herein such that an amount from about 1 ng of organic selenium/selenomethionine per kg to about 100 ng of organic selenium/selenomethionine per kg (e.g., from about 1 ng of organic selenium/selenomethionine per kg to about 80 ng of organic selenium/selenomethionine per kg, from about 1 ng of organic selenium/selenomethionine per kg to about 70 ng of organic selenium/selenomethionine per kg, from about 5 ng of organic selenium/selenomethionine per kg to about 100 ng of organic selenium/selenomethionine per kg, or from about 5 ng of organic selenium/selenomethionine per kg to about 75 ng of organic selenium/selenomethionine per kg) is administered to the mammal (e.g., a human).

Examples of inorganic nitrates that can be included in a xyloglucan preparation provided herein include, without limitation, sodium nitrate and potassium nitrate. The inorganic nitrate can be a low, non-toxic concentration of inorganic nitrate. In some embodiments, the inorganic nitrate is a low, non-toxic concentration of sodium nitrate. Any appropriate amount of inorganic nitrate can be used to formulate a xyloglucan preparation. For example, when treating cells *in vitro,* the cells can be exposed to a xyloglucan preparation provided herein such that an amount from about 0.5 µg of inorganic nitrate per mL to about 50 µg of inorganic nitrate per mL (e.g., from about 0.5 µg of inorganic nitrate per mL to about 40 µg of inorganic nitrate per mL, from about 0.5 µg of inorganic nitrate per mL to about 30 µg of inorganic nitrate per mL, from about 1 µg of inorganic nitrate per mL to about 50 µg of inorganic nitrate per mL, or from about 1µg of inorganic nitrate per mL to about 25 µg of inorganic nitrate per mL) is achieved. When treating cells *in vivo,* a mammal (e.g., a human) can be administered a xyloglucan preparation provided herein such that an amount from about 5 µg of inorganic nitrate per kg to about 500 µg of inorganic nitrate per kg (e.g., from about 5 µg of inorganic nitrate per kg to about 400 µg of inorganic nitrate per kg, from about 5 µg of inorganic nitrate per kg to about 300 µg of inorganic nitrate per kg, from about 10 µg of inorganic nitrate per kg to about 500 µg of inorganic nitrate per kg, or from about 25 µg of inorganic nitrate per kg to about 350 µg of inorganic nitrate per kg) is administered to the mammal (e.g., a human).

L-arginine can be included within a xyloglucan preparation provided herein to promote a cell's ability to release nitric oxide via nitric oxide synthesis from L-arginine metabolism. Any appropriate amount of L-arginine can be used to formulate a xyloglucan preparation. For example, when treating cells *in vitro,* the cells can be exposed to a xyloglucan preparation provided herein such that an amount from about 0.1 µg of L-arginine per mL to about 10 µg of L-arginine per mL (e.g., from about 0.1 µg of L-arginine per mL to about 7.5 µg of L-arginine per mL, from about 0.1 µg of L-arginine per mL to about 5 µg of L-arginine per mL, from about 0.5 µg of L-arginine per mL to about 10 µg of L-arginine per mL, or from about 0.5 µg of L-arginine per mL to about 7.5 µg of L-arginine per mL) is achieved. When treating cells *in vivo,* a mammal (e.g., a human) can be administered a xyloglucan preparation provided herein such that an amount from about 10 µg of L-arginine per kg to about 1000 µg of L-arginine per kg (e.g., from about 10 µg of L-arginine per kg to about 750 µg of L-arginine per kg, from about 10 µg of L-arginine per kg to about 500 µg of L-arginine per kg, from about 50 µg of L-arginine per kg to about 1000 µg of L-arginine per kg, or from about 25 µg of L-arginine per kg to about 750 µg of L-arginine per kg) is administered to the mammal (e.g., a human).

Calcium sources such as calcium citrate or CaCO₃ can be added to help facilitate the metabolism of L-arginine into nitric oxide via a calcium-dependent constitutive nitric oxide synthase. To reduce acid reflux problems in oral applications, CaCO₃ can be used as a calcium source. When treating cells *in vitro,* the cells can be exposed to a xyloglucan preparation provided herein such that an amount from about 0.05 µg of a calcium source per mL to about 5 µg of a calcium source per mL (e.g., from about 0.05 µg of a calcium source per mL to about 4 µg of a calcium source per mL, from about 0.05 µg of a calcium source per mL to about 3 µg of a calcium source per mL, from about 0.1 µg of a calcium source per mL to about 5 µg of a calcium source per mL, from about 0.25 µg of a calcium source per mL to about 5 µg of a calcium source per mL, or from about 0.1 µg of a calcium source per mL to about 2.5 µg of a calcium source per mL) is achieved. When treating cells *in vivo,* a mammal (e.g., a human) can be administered a xyloglucan preparation provided herein such that an amount from about 5 µg of a calcium source per kg to about 200 µg of a calcium source per kg (e.g., from about 5 µg of a calcium source per kg to about 180 µg of a calcium source per kg, from about 5 µg of a calcium source per kg to about 170 µg of a calcium source per kg, from about 10 µg of a calcium source per kg to about 200 µg of a calcium source per kg, from about 50 µg of a calcium source per kg to about 200 µg of a calcium source per kg, or from about 10 µg of a calcium source per kg to about 175 µg of a calcium source per kg) is administered to the mammal (e.g., a human).

Any appropriate method can be used to obtain an enzyme cofactor targeting mitochondrial complex 1 activity, a B complex vitamin, ubiquinone, selenium, inorganic nitrate, arginine, or calcium. In some cases, these components can be obtained using common chemical extraction, isolation, or synthesis techniques. In some cases, these components can be obtained from commercial vendors. For example, organic selenium and inorganic nitrate can be ordered from Sigma, Inc.

In some cases, a xyloglucan preparation provided herein can be formulated as a pharmaceutical composition and/or a nutritional supplement (e.g., a "nutraceutical"). For example, a xyloglucan preparation can be formulated to contain a pharmaceutically and/or nutritionally acceptable carrier for administration to a mammal. Examples of such carriers include, without limitation, sterile aqueous or non-aqueous solutions, solvents, suspensions, and emulsions. Examples of non-aqueous solvents include, without limitation, propylene glycol, polyethylene glycol, vegetable oils, and organic esters. Aqueous carriers include, without limitation, water, alcohol, saline, and buffered solutions. Acceptable carriers also can include physiologically acceptable aqueous vehicles (e.g., physiological saline) or other carriers used for oral administration.

An acceptable aqueous vehicle can be, for example, any liquid solution that is capable of dissolving a xyloglucan material obtained from a potato and that is not toxic to the particular mammal (e.g., a human) receiving the composition. Examples of acceptable aqueous vehicles include, without limitation, saline, water, and acetic acid. Typically, acceptable aqueous vehicles are sterile. An acceptable solid vehicle can be formulated such that a xyloglucan preparation is suitable for oral administration. The dose supplied by each capsule or tablet can vary since an effective amount can be reached by administrating either one or multiple capsules or tablets. Any appropriate pharmaceutically or nutritionally acceptable material such as gelatin and cellulose derivatives can be used as an acceptable solid vehicle. In addition, an acceptable solid vehicle can be a solid carrier including, without limitation, starch, sugar, or bentonite. Further, a tablet or pill formulation of a xyloglucan preparation can include solid carriers or lubricants. In some cases, a formulation of a xyloglucan preparation provided herein can be formulated for controlled release. For example, a xyloglucan preparation can be formulated to be located within an inner core of a capsule, tablet, gelcap, or pill with an outer coating that protects the inner core material from, for example, stomach acids. In some cases, the controlled release formulation can deliver the xyloglucan preparation to locations of the digestive tract past the stomach with little or no xyloglucan material being delivered to the stomach.

After a xyloglucan preparation provided herein is used to increase the level of expression of mitochondrial respiratory complex 1 polypeptides, to increase the level of expression of mitochondrial trafficking polypeptides, and/or to increase the level of mitochondrial activity within cells whether treated with the xyloglucan preparation *in vitro* or *in vivo,* the cells can be collected from tissue culture or harvested from a mammal. Once the xyloglucan-treated cells are collected, mitochondria can be isolated from those cells to obtain a mitochondrial preparation. Any appropriate method can be used to isolate intact and viable mitochondria from cells. For example, cell lysis and centrifugation methods can be used to isolate mitochondria from cells. In some cases, commercial kits such as the MITOISO2 kit (Sigma-Aldrich) can be used to isolate mitochondria from cells. In some cases, a filtration technique such as those described elsewhere (International Patent Application Publication No. WO 2015/192020) can be used to isolate mitochondria from cells.

As described herein, mitochondria isolated from cells treated with a xyloglucan preparation provided herein can exhibit more activity than mitochondria isolated from comparable cells not treated with a xyloglucan preparation. Any appropriate method can be used to assess the activity of mitochondria. For example, dyes and stains can be used to assess mitochondrial activity. Examples of such stains and dyes include, without limitation, MitoTracker® probes (ThermoFisher) such as MitoTracker Orange CMTMRos (ThermoFisher; catalog # M7510), MitoTracker Red CMXRos (ThermoFisher; catalog # M7512), and MitoTracker Red FM (ThermoFisher; catalog # M22425), rhodamines such as rhodamine 6G and rhodamine 123, tetramethylrosamine, RedoxSensor™ Red CC-1 (2,3,4,5,6-pentafluorotetramethyldihydrorosamine; ThermoFisher; catalog # R14060), JC-1 probes (5,5',6,6'-tetrachloro-1,1',3,3'-tetraethylbenzimidazolylcarbocyanine iodide; ThermoFisher; catalog # T3168), JC-9 (3,3'-dimethyl-(3-naphthoxazolium iodide; ThermoFisher; catalog # D22421), and DASPMI (4-Di-1-ASP; ThermoFisher; catalog # D288).

In some cases, mitochondria of a mitochondrial preparation provided herein can be used as obtained from cells treated with a xyloglucan preparation. For example, mitochondria isolated from cells treated with a xyloglucan preparation can be used to create a mitochondrial preparation without any further manipulation. In some cases, mitochondria of a mitochondrial preparation provided herein can be used as obtained from cells treated with a xyloglucan preparation and can be the sole active ingredient of the mitochondrial preparation.

In some cases, a mitochondrial preparation provided herein can be formulated to include one or more other ingredients in addition to mitochondria isolated from cells treated with a xyloglucan preparation. For example, a mitochondrial preparation provided herein can be formulated to include one or more (e.g., one, two, three, four, five, or more) enzyme cofactors targeting mitochondrial complex 1 activity, one or more (e.g., one, two, three, or more) B complex vitamins, ubiquinone, selenium, inorganic nitrate, arginine, calcium, or a combination thereof. In some cases, a mitochondrial preparation provided herein can be formulated to include a xyloglucan preparation provided herein in addition to mitochondria isolated from cells treated with a xyloglucan preparation provided herein.

A mitochondrial preparation provided herein can be administered to a mammal (e.g., a human) to improve cellular function and/or to reduce a symptom of a disease or disorder (e.g., a disease or disorder associated with mitochondrial dysfunction or deficiency). For example, a mitochondrial preparation provided herein can be administered to a mammal to improve cellular functions such as glucose metabolism and transport, oxidative phosphorylation, electron transport activity, intracellular redox potential, mitochondrial biogenesis and trafficking, gene transcription and mRNA editing, protein translation and intracellular signaling. In some cases, a mitochondrial preparation provided herein can be administered to a mammal to treat a disease or disorder (e.g., a disease or disorder associated with mitochondrial dysfunction or deficiency) and/or to reduce a symptom of a disease or disorder (e.g., a disease or disorder associated with mitochondrial dysfunction or deficiency). Examples of diseases or disorders associated with mitochondrial dysfunction or deficiency that can be treated as described herein include, without limitation, Type II diabetes, steatotic liver disease, cardiovascular disease, renal disease, metastatic lung, pancreatic, and breast cancers, atherosclerosis, rheumatoid arthritis, autoimmune disease, Alzheimer's Disease, Parkinson's Disease, mitochondrial myopathy, Leigh syndrome, and Friedreich's ataxia. A mitochondrial preparation provided herein can be administered to any appropriate type of mammal to improve cellular function and/or to reduce a symptom of a disease or disorder including, without limitation, rats, mice, dogs, cats, horses, bovine species, goats, pigs, monkeys, chickens, turkeys, ducks, or humans.

In some cases, a mitochondrial preparation can be obtained from cells (e.g., white blood cells) obtained from a mammal to be treated (e.g., a particular human to be treated) and then that mitochondrial preparation can be administered to that particular mammal as described herein. For example, a mammal to be treated as described herein can be administered a mitochondrial preparation containing autologous mitochondria. In some cases, a mammal to be treated as described herein can be administered a mitochondrial preparation containing xenogeneic or allogeneic mitochondria.

Any appropriate method can be used to administer a mitochondrial preparation provided herein to a mammal. For example, a mitochondrial preparation provided herein can be directly injected into target tissue (e.g., myocardial tissue, cardiovascular tissue, peripheral vascular tissue, hepatic tissue, or renal tissue) of a mammal being treated. In some cases, when treating myocardial ischemia, a mitochondrial preparation provided herein can be directly injected into the ischemic myocardium. In some cases, when treating diabetes (e.g., Type II diabetes), a mitochondrial preparation provided herein can be administered systemically (e.g., via intravenous administration). Other routes of administration for administering a mitochondrial preparation provided herein to a mammal include, without limitation, intraperitoneal administrations, intracranial administrations, intranasal administrations, intramuscular administrations, intradermal administrations, or intraarticular administrations.

After administering a mitochondrial preparation provided herein to a mammal, the mitochondria can provide tissues and cells of the mammal with several benefits both when present extracellularly and intracellularly within the mammal. For example, when administered to a mammal and present extracellularly within that mammal, the intact and viable mitochondria administered to the mammal as described herein can provide cells and tissue with ATP and can stimulate the expression of polypeptides involved mitochondrial pathways. When administered to a mammal and internalized into cells within that mammal, the intact and viable mitochondria administered to the mammal as described herein can provide those cells with increased ATP production and a source of new, exogenously added mitochondrial DNA.

A mitochondrial preparation provided herein can have any appropriate concentration of mitochondria for administration to a mammal. For example, a mitochondrial preparation provided herein for administration to a mammal (e.g., a human) can contain from about 1 x 10³ to about 1 x 10⁹ mitochondria (e.g., from about 1 x 10⁴ to about 1 x 10⁹ mitochondria, from about 1 x 10⁵ to about 1 x 10⁹ mitochondria, from about 1 x 10³ to about 1 x 10⁸ mitochondria, from about 1 x 10³ to about 1×10⁷ mitochondria, from about 1 x 10⁴ to about 1 x 10⁸ mitochondria, from about 1 x 10⁵ to about 1 x 10⁸ mitochondria, or from about 1 x 10⁶ to about 1 x 10⁸ mitochondria) per mL. Any appropriate volume of a mitochondrial preparation provided herein can be administered to a mammal. For example, from about 10 mL to about 100 mL (e.g., from about 10 mL to about 80 mL, from about 10 mL to about 70 mL, from about 10 mL to about 100 mL, from about 15 mL to about 100 mL, or from about 15 mL to about 75 mL) of a mitochondrial preparation containing from about 1 x 10⁶ to about 1×10⁸ mitochondria per mL can be administered to a mammal.

In some cases, mitochondria of a mitochondrial preparation provided herein can be packaged within liposomes and administered to a mammal as liposome-packaged mitochondria. Any appropriate method can be used to package mitochondria into liposomes such as those described elsewhere (Durazo and Kompella, Mitochondrion, 12(2): 190-201 (2012)). In some cases, mitochondria of a mitochondrial preparation provided herein can be free of liposomes.

In some cases, mitochondria of a mitochondrial preparation provided herein can be delivered to cells *in vitro* for intemalization as described elsewhere (Yang and Koob, Nucl. Acid Res., 40(19):e148 (2012)). Any appropriate cell type can be exposed to a mitochondrial preparation provided herein to deliver exogenous mitochondria to those cells. For example, myocardial cells, cardiovascular cells, nerve cells, peripheral vascular endothelial cells, hepatic cells, neurons, and renal cells can be exposed to a mitochondrial preparation provided herein *in vitro* to deliver mitochondria from that mitochondrial preparation to those cells. In some cases, after exposing cells to a mitochondrial preparation provided herein *in vitro,* those cells can be administered to a mammal. In some cases, the cells receiving mitochondria from a mitochondrial preparation provided herein and being administered to a mammal can be from that same mammal. For example, cells can be obtained from a particular mammal to be treated, exposed to a mitochondrial preparation provided herein (e.g., a mitochondrial preparation containing autologous, allogeneic, or xenogeneic mitochondria), and reintroduced into that same particular mammal. In some cases, cells not obtained from a particular mammal to be treated can be exposed to a mitochondrial preparation provided herein and introduced into that particular mammal. In some cases, the cells receiving autologous, allogeneic, or xenogeneic mitochondria from a mitochondrial preparation provided herein and being administered to a mammal can be autologous, allogeneic, or xenogeneic cells to that mammal.

The methods for treating a disease or disorder (e.g., a disease or disorder associated with mitochondrial dysfunction or deficiency) using a mitochondrial preparation as described herein can include identifying the mammal as having a disease or disorder associated with mitochondrial dysfunction or deficiency. For example, a mammal can be identified as having cardiovascular disease using Real time fNMR and PET imaging techniques and then the identified mammal can be administered a mitochondrial preparation as described herein or cells treated with a mitochondrial preparation as described herein.

Any appropriate amount of a composition containing a mitochondrial preparation provided herein or containing cells exposed to a mitochondrial preparation provided herein can be used to treat a mammal having a disease or disorder (e.g., a disease or disorder associated with mitochondrial dysfunction or deficiency). For example, a composition containing a mitochondrial preparation can be formulated to contain from about 50 mg to about 500 mg (e.g., from about 50 mg to about 480 mg, from about 50 mg to about 470 mg, from about 60 mg to about 500 mg, from about 70 mg to about 500 mg, or from about 75 mg to about 450 mg) of a mitochondrial preparation. In some cases, an effective amount can be any amount that reduces, prevents, or eliminates a symptom of a disease or disorder upon administration to a mammal without producing significant toxicity to the mammal. Various factors can influence the actual amount used for a particular application. For example, the frequency of administration, duration of treatment, combination of other agents, site of administration, stage of disease (if present), and the anatomical configuration of the treated area may require an increase or decrease in the actual amount administered.

Any appropriate frequency of administration of a composition containing a mitochondrial preparation provided herein or containing cells exposed to a mitochondrial preparation provided herein can be used to treat a mammal having a disease or disorder (e.g., a disease or disorder associated with mitochondrial dysfunction or deficiency). For example, the frequency of administration can be from about 2 times a day to about 60 times a month. In addition, the frequency of administration can remain constant or can be variable during the duration of treatment. In some cases, an effective frequency can be any frequency that reduces, prevents, or eliminates a symptom of a disease or disorder upon administration to a mammal without producing significant toxicity to the mammal. As with the amount administered, various factors can influence the actual frequency of administration used for a particular application. For example, the amount, duration of treatment, combination of agents, site of administration, stage of disease (if present), and the anatomical configuration of the treated area may require an increase or decrease in administration frequency.

Any appropriate duration of administration of a composition containing a mitochondrial preparation provided herein or containing cells exposed to a mitochondrial preparation provided herein can be used to treat a mammal having a disease or disorder (e.g., a disease or disorder associated with mitochondrial dysfunction or deficiency). For example, a duration of administration can be a week, month, three months, six months, nine months, a year, two years, three years, or longer. In some cases, an effective duration can be any duration that reduces, prevents, or eliminates a symptom of a disease or disorder upon administration to a mammal without producing significant toxicity to the mammal. Multiple factors can influence the actual duration used for a particular treatment regimen. For example, an effective duration can vary with the frequency of administration, the amount administered, combination of multiple agents, site of administration, state of disease (if present), and anatomical configuration of the treated area.

While not being limited to any particular mode of action, a xyloglucan preparation provided herein can be used to increase or decrease the expression of particular polypeptides within cells. For example, a xyloglucan preparation provided herein can be used to increase expression of Ndufa8 (NADH:ubiquinone oxidoreductase subunit A8), Ndufa10 (NADH:ubiquinone oxidoreductase subunit A10), Ndufall (NADH:ubiquinone oxidoreductase subunit A11), Ndufa12 (NADH:ubiquinone oxidoreductase subunit A12), Ndufa13 (NADH:ubiquinone oxidoreductase subunit A13), Ndufb4 (NADH:ubiquinone oxidoreductase subunit B4), Ndufb5 (NADH:ubiquinone oxidoreductase subunit B5), Ndufb7 (NADH:ubiquinone oxidoreductase subunit B7), Ndufb9 (NADH:ubiquinone oxidoreductase subunit B9), Ndufb10 (NADH:ubiquinone oxidoreductase subunit B10), Ndufs4 (NADH:ubiquinone oxidoreductase core subunit S4), Ndufs6 (NADH:ubiquinone oxidoreductase core subunit S6), Ndufs7 (NADH:ubiquinone oxidoreductase core subunit S7), Fez2 (fasciculation and elongation protein zeta 2), Pin1 (peptidylprolyl cis/trans isomerase, NIMA-interacting 1), Ranbpl (RAN binding protein 1), Rhot2 (ras homolog family member T2), or a combination thereof.

In some cases, a xyloglucan preparation provided herein can be used to increase expression of aNdufa8 polypeptide by at least about 1.5 fold (e.g., by about 1.7 fold). In some cases, a xyloglucan preparation provided herein can be used to increase expression of a Ndufa10 polypeptide by at least about 1.2 fold (e.g., by about 1.4 fold). In some cases, a xyloglucan preparation provided herein can be used to increase expression of a Ndufa11 polypeptide by at least about 1.8 fold (e.g., by about 2.0 fold). In some cases, a xyloglucan preparation provided herein can be used to increase expression of a Ndufa12 polypeptide by at least about 1.5 fold (e.g., by about 1.7 fold). In some cases, a xyloglucan preparation provided herein can be used to increase expression of a Ndufa13 polypeptide by at least about 1.8 fold (e.g., by about 2.0 fold). In some cases, a xyloglucan preparation provided herein can be used to increase expression of a Ndufb4 polypeptide by at least about 1.5 fold (e.g., by about 1.7 fold). In some cases, a xyloglucan preparation provided herein can be used to increase expression of a Ndufb5 polypeptide by at least about 1.1 fold (e.g., by about 1.3 fold). In some cases, a xyloglucan preparation provided herein can be used to increase expression of a Ndufb7 polypeptide by at least about 1.3 fold (e.g., by about 1.5 fold). In some cases, a xyloglucan preparation provided herein can be used to increase expression of a Ndufb9 polypeptide by at least about 1.5 fold (e.g., by about 1.8 fold). In some cases, a xyloglucan preparation provided herein can be used to increase expression of a Ndufb10 polypeptide by at least about 1.3 fold (e.g., by about 1.5 fold). In some cases, a xyloglucan preparation provided herein can be used to increase expression of a Ndufs4 polypeptide by at least about 1.2 fold (e.g., by about 1.4 fold). In some cases, a xyloglucan preparation provided herein can be used to increase expression of a Ndufs6 polypeptide by at least about 1.7 fold (e.g., by about 1.9 fold). In some cases, a xyloglucan preparation provided herein can be used to increase expression of a Ndufs7 polypeptide by at least about 1.5 fold (e.g., by about 1.7 fold). In some cases, a xyloglucan preparation provided herein can be used to increase expression of a Fez2 polypeptide by at least about 1.2 fold (e.g., by about 1.4 fold). In some cases, a xyloglucan preparation provided herein can be used to increase expression of a Pinl polypeptide by at least about 1.0 fold (e.g., by about 1.2 fold). In some cases, a xyloglucan preparation provided herein can be used to increase expression of a Ranbpl polypeptide by at least about 1.0 fold (e.g., by about 1.2 fold). In some cases, a xyloglucan preparation provided herein can be used to increase expression of a Rhot2 polypeptide by at least about 1.2 fold (e.g., by about 1.4 fold).

In some cases, a xyloglucan preparation provided herein can be used to decrease expression of Trak1 (trafficking kinesin protein 1). In some cases, a xyloglucan preparation provided herein can be used to decrease expression of a Trak1 polypeptide by at least -7.0 fold (e.g., by about -7.7 fold).

A human Ndufa8 polypeptide can have an amino acid sequence as set forth in, for example, National Center for Biotechnology Information (NCBI) Accession No: NG 042279
(GI No. 1003701614). A human Ndufa10 polypeptide can have an amino acid sequence as set forth in, for example, NCBI Accession No: NG_031855 (GI No. 1046552732). A human Ndufa1 1 polypeptide can have an amino acid sequence as set forth in, for example, NCBI Accession No: NG_027808 (GI No. 308387358). A human Ndufa12 polypeptide can have an amino acid sequence as set forth in, for example, NCBI Accession No: NG_032672(GI No. 385719181). A human Ndufa13 polypeptide can have an amino acid sequence as set forth in, for example, NCBI Accession No: NG_013380 (GI No. 262359939). A human Ndufb4 polypeptide can have an amino acid sequence as set forth in, for example, NCBI Accession No: NM_001168331.1 (GI No. 270288779). A human Ndufb5 polypeptide can have an amino acid sequence as set forth in, for example, NCBI Accession No: NM_002492 (GI No. 316659417). A human Ndufb7 polypeptide can have an amino acid sequence as set forth in, for example, NCBI Accession No: NM_004146 (GI No. 316983134). A human Ndufb9 polypeptide can have an amino acid sequence as set forth in, for example, NCBI Accession No: NG_042266 (GI No. 906847380). A human Ndufb10 polypeptide can have an amino acid sequence as set forth in, for example, NCBI Accession No: NM_004548 (GI No. 161169039). A human Ndufs4 polypeptide can have an amino acid sequence as set forth in, for example, NCBI Accession No: NG_008200 (GI No. 194018396). A human Ndufs6 polypeptide can have an amino acid sequence as set forth in, for example, NCBI Accession No: NG_013354 (GI No. 262331551). A human Ndufs7 polypeptide can have an amino acid sequence as set forth in, for example, NCBI Accession No: NG_008283(GI No. 194394212). A human Fez2 polypeptide can have an amino acid sequence as set forth in, for example, NCBI Accession No: NM_005102.2 (GI No. 110349753). A human Pin1 polypeptide can have an amino acid sequence as set forth in, for example, NCBI Accession No: NG_029167
(GI No. 336455056). A human Ranbp1 polypeptide can have an amino acid sequence as set forth in, for example, NCBI Accession No: NM_001278639.1 (GI No. 520975476). A human Rhot2 polypeptide can have an amino acid sequence as set forth in, for example, NCBI Accession No: NG_031824 (GI No. 359279883). A human Trak1 polypeptide can have an amino acid sequence as set forth in, for example, NCBI Accession No: NM_001042646.2 (GI No. 388490360).

The invention will be further described in the following examples, which do not limit the scope of the invention described in the claims.

### EXAMPLES

### Example 1 - Producing improved mitochondria

### Methods and materials

### Extraction and purification of xyloglucan preparation

About 6 g of potato were homogenized with a Polytron homogenizer in 20 mL water in a 50 mL centrifuge tube and kept at room temperature for 1 hour. The homogenate was centrifuged at 4000 rpm for 10 minutes, and the supernatant fraction was reserved. 10 mL of the supernatant fraction was percolated through a Sep-Pak Plus C-18 cartridge previously activated with 10 mL 100% acetonitrile (ACN) followed by 10 mL 0.05% trifluoroacetic acid in water (TFA water). Following successive low ACN washes, semi-purified xyloglucan material was eluted in 10 mL 10% ACN in 0.05% TFA water. The eluent fraction was dried and reconstituted in 1 mL 0.05% TFA water for further purification via HPLC.

The reconstituted 10% ACN eluent fraction was subjected to HPLC purification using a Waters Xterra RP C18 column (4.6 x 150 mm) and Waters 2695 separations module with a photodiode array detector. HPLC purification involved using a shallow 20 minute gradient ranging from 0 to 2.5% in 0.05% TFA water at a flow rate of 0.5 mL/minute. Collection and HPLC re-purification of a major 198 nm UV absorbing peak at 3.5 minutes yielded a symmetrical HPLC peak containing highly purified xyloglucan material. The purified HPLC fraction of xyloglucan material was dried and reconstituted in phosphate buffered saline (PBS) for use in biological experiments.

### Experimental animals

Twenty-two 7-week old, male homozygous Zucker Diabetic Fatty rats (ZDF, Code: 370) and twenty-two 7-8 week old, male heterozygous Zucker Lean Control rats (ZLC, Code: 371) were purchased from Charles Rivers Laboratories (Wilmington, MA). The study animals were allowed an acclimation period of four days prior to baseline blood collections, at which time two extra animals from each strain were dropped from the study based on baseline body weight. The rats were housed two per cage and maintained in the Innovive caging system (San Diego, CA). Cages were monitored daily to ensure the Innovive system maintained 80 air changes per hour and positive pressure. Rat rooms were maintained at temperatures of 66-75°F and relative humidity between 30% and 70%. The rooms were lit by artificial light for 12 hours each day (7:00 am - 7:00 pm). Animals had free access to water and Purina 5008 rodent food (Waldschimdt's Town & Country Mart; Madison, WI) for the duration of the study except during fasting experiments.

### Xyloglucan formulation

Purified xyloglucan (10 mL stock solution at 5 mg/mL concentration) was stored at 4°C. The vehicle for the study was sterile water (Catalog number 002488, Butler Schein). Each week, the stock solution was diluted 1:100 in sterile water (0.05 mg/mL) and dispensed into daily aliquots. All vehicle and drug solutions were stored at 4°C and administered at room temperature daily by oral gavage (PO) in a volume of 1 mL/animal (0.15 mg/kg dose based on estimated body weight of 350 g).

### Dosing and grouping

Two types of rats were used for the study: homozygous obese ZDF diabetic animals and heterozygous ZLC littermates. The rats within the groups were then chosen at random and divided into groups of 7. Group 1 consisted of ZDF rats fed vehicle; Group 2 consisted of ZDF rats fed xyloglucan; Group 3 consisted of ZLC rats fed vehicle; and Group 4 consisted of ZLC rats fed xyloglucan. The vehicle was distilled water, and the xyloglucan was given daily each morning via oral gavage at a dosage of 0.05 mg per animal. The dose was usually given in 1 mL of water.

The study lasted for 28 days, and all animals were euthanized by isoflurane overdose and thoracotomy following the collection of fasted blood glucose data on day 28 of the study. Blood was collected via descending vena cava. Liver and abdominal fat were collected and weighed, and a portion of the left lateral liver lobe and abdominal fat were placed into individual histology cassettes and snap frozen in liquid nitrogen. General pathological observations were recorded.

### RNA isolation

Total RNA extracted from rat tissue samples was isolated and purified using the RNeasy mini kit (Qiagen, Valencia, CA). About 100 mg of tissue was resuspended in 1.8 mL of RLT lysis buffer (Qiagen) and homogenized with a polytron homogenizer for 30 seconds. Blood RNA was isolated using the PAX RNA kit (Qiagen).

### DNA microarray analyses

DNA microarray analyses were performed using a system provided by Agilent. Arrays included four arrays per chip (Agilent 4X44K chip). Total RNA was reverse transcribed (1000 ng) using T7 primers and labeled and transcribed using Cyanine-3 dye. Each array was hybridized with at least 1.65 µg of labeled cRNA at 65°C for 18 hours. Arrays were scanned using an Agilent array scanner. The microarray platform can determine a minimum of a 1.5 fold change in gene expression. Comparative fold changes in gene expressed were measured and statistically analyzed between the 4 Study Groups: homozygous ZDF rats fed xyloglucan for 28 days (Group 2) vs homozygous ZDF rats that were fed an equivalent amount of vehicle (Group 1) vs heterozygous ZLC lean rats fed xyloglucan for 28 days (Group 4) vs heterozygous ZLC lean rats fed an equivalent amount of vehicle for 28 days (Group 3). % Gene restoration calculations were based on the ratio of fold changes of ZDF rats fed xyloglucan to ZLC healthy rats fed xyloglucan.

### Assessing mitochondrial activity

HTB-11 cell grown at 70% confluency were treated for 24 hours with xyloglucan (65 µg/mL) or vehicle followed by a 30 minute incubation with rhodamine 6G at a final concentration of 2 µM. After extensive washing with phosphate buffered saline, fluorescent images indicating extensive and selective mitochondrial uptake and retention of rhodamine 6G were captured at 20 ms exposure using a Nikon Ti Epi Fluorescence Module with High Intensity LED illumination System, including filters sets for FITC, GFP, and RFP.

### Purification offunctionally competent mitochondria from whole blood

Mitochondrial preparations were obtained from 2x10⁷ white blood cells (WBC). Heparinized whole blood samples (50 mL per donor) were obtained and immediately separated using 1-Step Polymorphs (Accurate Chemical and Scientific Corporation, Westbury, NY) gradient medium. Five mL of blood was layered over 5 mL of polymorphs in a 14 mL round-bottom tube and then centrifuged for 35 minutes at 500 x g in a swinging-bucket rotor at 18°C. After centrifugation, the bands containing polymorphonuclear cells (PMN) and mononuclear cells (MN) were harvested using glass pipettes and transferred into 15 mL centrifuge tubes and then washed with 10 mL phosphate buffered saline (PBS) (Invitrogen, Carlsbad, CA). Cells were collected after centrifugation for 10 minutes at 400 x g. Cells were incubated at 37°C in RPMI-1640 media supplemented with 10% fetal bovine serum (Invitrogen) and allowed to recover for 60 minutes.

WBC were washed and pelleted with cold (4°C) PBS prior to mitochondrial isolation. The mitochondria were isolated using a mitochondria isolation kit for cultured cells (MITOISO2; Sigma-Aldrich). This method resulted in an enriched and intact mitochondrial fraction from cells. The detergent lysis method was used and required 650 µL of lysis buffer. The lysed cells were centrifuged at 700g for 10 minutes, and the resulting supernatant was centrifuged at 3000g for 15 minutes. This next supernatant contained the cytosolic fraction and was transferred to another tube. The mitochondrial pellet was washed with 500 µL of the provided wash reagent. The washed mitochondria were centrifuged for 5 minutes at 12000g. The supernatant was discarded, and the pellet was re-suspended in 1 mL of storage buffer.

For protein quantification, mitochondria were lysed by vortexing for 1 minute in 100 µL of 2% CHAPS in Tris buffered saline (25 mM Tris, 0.15 M NaCl; pH 7.2) and centrifuged at 12000g for 2 minutes. The supernatant was analyzed using the Bio-Rad protein assay and a microplate reader (Microplate Manager® 4.0).

### Validation of mitochondrial purity

For Western Blots, purified proteins (∼1.4 µg per preparation) were combined with 10 µL of Bolt™ LDS sample buffer (4x) and 30 µL deionized water. Samples were denatured at 90°C for 10 minutes and separated via protein electrophoresis on a NuPAGE® Novex® 4-12% Bis-Tris Mini-gel at 165 V for 28 minutes. Following electrophoresis, the samples were transferred onto a nitrocellulose membrane using the iBlot® Gel Transfer Device system (Invitrogen). Thereafter, using the iBlot® Gel Device for Western Detection (Invitrogen), the membrane was subjected to a 3-step program consisting of a blocking step, primary antibody step, and a secondary antibody step. The primary antibody, mouse anti-Cytochrome C monoclonal, was obtained from Life Technologies (Invitrogen Cat. No. 33-8500) and diluted 1:1000 with blocking buffer. The secondary antibody was diluted 1:2000 with blocking buffer. The membrane was then sequentially washed three times in 1X wash solution and rinsed in deionized water before incubation at room temperature. Finally, with gentle rocking at room temperature, the blot was developed using Chromogen (BCIP/NBT) as a chromogenic substrate.

### Validation of the mitochondrial transfer efficiency via microscopy and real time PCR

The transfer of isolated mitochondria from donor cells to recipient cells was performed immediately after isolation. The mitochondria were incubated for 24 hours with 2 mL of media in 6 well plates with 1x10⁵ recipient cells. For the viability and functional assays, the cells were incubated in 96 well plates.

Mitochondrial DNA (mtDNA) content and nuclear DNA content were compared using real time PCR. DNA was isolated and purified using the QIAamp DNA mini kit (Qiagen). Real-time PCR was performed with human NADH dehydrogenase subunit 1 (ND1) detector set (Hs02596873_s1) for the mtDNA and tyrosine 3-monooxygenase/tryptophan 5-monooxygenase activation protein, zeta polypeptide (YWHAZ) detector set (Hs03044281_g1) for nuclear DNA (ThermoFisher Scientific). Using 1 µL of DNA per reaction, all samples were analyzed in triplicate. The real-time PCR master mix included 25 µL 2X universal master mix, 2.5 µL 20X detector set (with the primer and probe), and 21.5 µL of water. PCR was performed using an Applied Biosystems Quant Studio 7 Flex system. The thermocycler conditions included denaturation at 95°C for 15 seconds with combined annealing and extension at 60°C for 60 seconds. Forty cycles of PCR were preceded by 95°C for 10 minutes. Relative quantities of mtDNA was assessed using the average ratio of mtDNA to nuclear DNA in donor cells and recipient cells prior to transplantation compared to the ratio of mtDNA to nuclear DNA in recipient cells after transplantation. Visualization of GFP or DsRed2FP labeled mitochondria in recipient cells was performed using confocal microscopy.

### Assay of cellular viability and mitochondrial function

Viability of recipient cells was determined using a resazurin-based assay kit as described elsewhere (Kitani et al., J. Cell. Mol. Med., 18(8):1694-703 (2014)) and oxygen consumption rates, extracellular acidification rates, and ATP production were quantified using Seahorse XF instruments. The instrumentation provides rapid analysis of mitochondrial respiration and glycolysis in live cells via solid-state sensors to simultaneously measure both oxygen consumption rate (OCR) and extracellular acidification rate (ECAR). All instrumentation platforms support multiple administrations of pharmacological and metabolic agents to live cells contained in multiple wells.

### Results

*In vivo* administration of purified xyloglucan to ZDF rats (n=7) vs vehicle-fed control ZDF rats (n=7) engendered statistically significant enhanced expression of 13 high profile candidate genes that were involved in synergistic enhancement of mitochondrial complex 1 activity (Table 1). In dramatic fashion, *in vivo* xyloglucan treatment restored the aberrant gene expression of the homozygous ZDF rats to near normal expression levels observed in healthy ZLC rats with a high degree of statistical significance (80-110% restoration of normative gene function).

**Table 1. Enhanced expression of nuclear-encoded mitochondrial complex 1 genes, as determined in blood samples of ZDF rats treated with xyloglucan and compared to vehicle-fed ZDF rats.**

| **Gene Symbol** | **Gene Name** | **Fold change (xyloglucan)** | ***p* value** | **% Restored to lean control values** |
|---|---|---|---|---|
| **Ndufa10** | NADH:ubiquinone oxidoreductase subunit A8 | 1.4 | 0.035 | 83 |
| **Ndufa11** | NADH:ubiquinone oxidoreductase subunit A11 | 2.0 | 0.008 | 130 |
| **Ndufa12** | NADH:ubiquinone oxidoreductase subunit A12 | 1.7 | 0.016 | 129 |
| **Ndufa13** | NADH:ubiquinone oxidoreductase subunit A13 | 2.0 | 0.008 | 117 |
| **Ndufa8** | NADH:ubiquinone oxidoreductase subunit A8 | 1.7 | 0.004 | 148 |
| **Ndufb10** | NADH:ubiquinone oxidoreductase subunit B10 | 1.5 | 0.031 | 90 |
| **Ndufb4** | NADH:ubiquinone oxidoreductase subunit B4 | 1.7 | 0.013 | 117 |
| **Ndufb5** | NADH:ubiquinone oxidoreductase subunit B5 | 1.3 | 0.055 | 83 |
| **Ndufb7** | NADH:ubiquinone oxidoreductase subunit B7 | 1.5 | 0.032 | 95 |
| **Ndufb9** | NADH:ubiquinone oxidoreductase subunit B9 | 1.8 | 0.008 | 110 |
| **Ndufs4** | NADH:ubiquinone oxidoreductase core subunit S4 | 1.4 | 0.043 | 75 |
| **Ndufs6** | NADH:ubiquinone oxidoreductase core subunit S6 | 1.9 | 0.008 | 129 |
| **Ndufs7** | NADH:ubiquinone oxidoreductase core subunit S7 | 1.7 | 0.020 | 103 |

*In vivo* administration of purified xyloglucan to ZDF rats (n=7) vs vehicle control ZDF rats (n=7) engendered statistically significant enhanced expression of high profile mitochondrial trafficking genes and marked reduction of Trak1, as monitored in blood samples of ZDF rats vs vehicle-fed control ZDF rats (Table 2).

**Table 2. Enhanced expression of nuclear-encoded mitochondrial trafficking genes and marked reduction of Trak1 expression, as determined in blood samples.**

| **Gene Symbol** | **Gene Name** | **Fold change (xyloglucan)** | ***p* value** | **% Restored to lean control values** |
|---|---|---|---|---|
| **Fez2** | fasciculation and elongation protein zeta 2 | 1.4 | 0.0017 | 98 |
| **Pin1** | peptidylprolyl cis/trans isomerase, NIMA-interacting 1 | 1.2 | 0.0208 | 112 |
| **Ranbp1** | RAN binding protein 1 | 1.2 | 0.0232 | 72 |
| **Rhot2** | ras homolog family member T2 | 1.4 | 0.0099 | 118 |
| **Trak1** | trafficking kinesin protein 1 | -7.7 | 0.0017 | 99 |

The observed changes in complex 1 gene expression were validated utilizing an *in vitro* cellular model employing HTB-11 cells in conjunction with semi-quantitative labeling of functionally active mitochondria by the well characterized cationic dye rhodamine 6G. Classic biochemical studies have demonstrated an inhibitory effect of rhodamine 6G administration on mitochondrial complex 1 proton pump activities and FIFO ATPase-mediated ATP biosynthesis in isolated respiring rat liver mitochondria (Higuti et al., Biochim. Biophys. Acta, 593(2):463-7 (1980) and Bunting, Biophys. Chem., 42(2): 163-75 (1992)). Rhodamine 6G also was established as a highly reliable and highly selective semi-quantitative fluorescent probe of mitochondrial activity in living cells *in vitro* (Dietzmann et al., Exp. Pathol., 31(1):47-51 (1987)) and as a potentially invaluable PET ligand for *in vivo* imaging of human mitochondria within the cardiovascular system (Bartholoma et al., Nucl. Med. Biol., 42(10):796-803 (2015)).

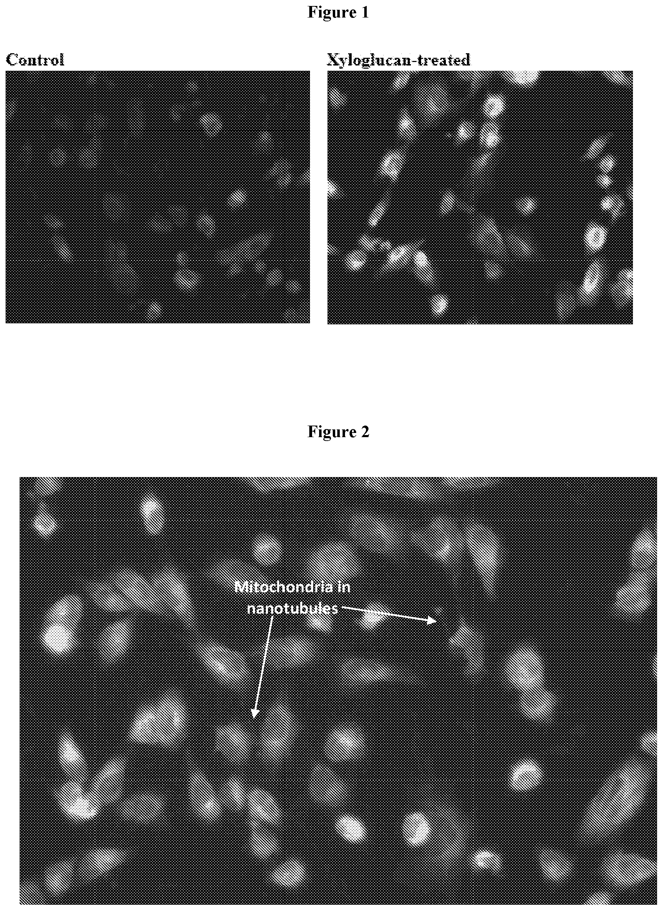

A dramatic 6.4 fold enhancement in the intensity of fluorescent mitochondria was observed in xyloglucan-treated HTB-11 cells maintained in culture for 24 hours as compared to vehicle-treated control cells (Figure 1). Based on three independently performed experiments, an average fold change of 6.4 in fluorescent intensity was observed in cultures treated with xyloglucan as compared to vehicle control cultures. The cells counted in the field of view for the control (n=138) had an average intensity per cell of 7.5 units, while the xyloglucan-treated cells in the field (n=192) had an average intensity per cell of 50.5 units. The average cell area for the control cells was 89 µm², and the average cell area for the xyloglucan-treated cells was 70 µm².

In addition, an enhanced expression of mitochondria-containing tunneling nanotubes was observed in xyloglucan-treated HTB-11 cells maintained in culture for 24 hours (Figure 2). Arrows indicate the presence of fluorescent mitochondria within tunneling nanotubes.

Taken together, these results demonstrate that xyloglucan can be used to obtain optimally functional mitochondrial preparations from mammalian blood via markedly enhanced coordinate gene expression of key subunits of mitochondrial respiratory Complex 1 and mitochondrial trafficking proteins. In particular, these results demonstrate the ability of *in vivo* administration of a xyloglucan provided herein to entrain coordinate enhancement of 15 subunits of Complex 1 in concert with requisite mitochondrial trafficking/adaptor proteins in blood leukocytes. These methods can be used to promote the expression of a highly abundant, easily purifiable, pool of optimally functional mitochondria from peripheral blood. These enhanced mitochondria can be used for mitochondria replacement therapy of multiple medical indications.

## Claims

1. A composition comprising potato xyloglucan material and viable mitochondria separated from cells.

2. The composition of claim 1, wherein said potato xyloglucan material was obtained from raw potato.

3. The composition of claim 1, wherein said cells are mammalian cells.

4. The composition of claim 3, wherein said cells are human cells.

5. The composition of claim 1, wherein said composition comprises organic selenium.

6. The composition of claim 1, wherein said composition comprises an inorganic nitrate.

## Patentansprüche

1. Zusammensetzung, umfassend Kartoffel-Xyloglucanmaterial und lebensfähige Mitochondrien, die von Zellen getrennt sind.

2. Zusammensetzung nach Anspruch 1, wobei das Kartoffel-Xyloglucanmaterial aus Rohkartoffel gewonnen wurde.

3. Zusammensetzung nach Anspruch 1, wobei es ich bei den Zellen um Säugerzellen handelt.

4. Zusammensetzung nach Anspruch 3, wobei es ich bei den Zellen um menschliche Zellen handelt.

5. Zusammensetzung nach Anspruch 1, wobei die Zusammensetzung organisches Selen umfasst.

6. Zusammensetzung nach Anspruch 1, wobei die Zusammensetzung ein anorganisches Nitrat umfasst.

## Revendications

1. Composition comprenant un matériau de xyloglucane de pomme de terre et des mitochondries viables séparées de cellules.

2. Composition selon la revendication 1, ledit matériau de xyloglucane de pomme de terre étant obtenu à partir de pomme de terre crue.

3. Composition selon la revendication 1, lesdites cellules étant des cellules mammifères.

4. Composition selon la revendication 3, lesdites cellules étant des cellules humaines.

5. Composition selon la revendication 1, ladite composition comprenant du sélénium organique.

6. Composition selon la revendication 1, ladite composition comprenant un nitrate inorganique.
